# EUROPEAN PATENT SPECIFICATION

(11) **EP 0 555 510 B1**
(45) Date of publication and mention of the grant of the patent: **29.09.1999**
(21) Application number: 92102531.8
(22) Date of filing: 14.02.1992
(51) Int. Cl.: A61N 1/30

(54) **Electrical transdermal drug applicator with counteractor**
Elektrisches Gerät zur transdermalen Verabreichung von Medikamenten mit einem entgegenwirkenden Produkt
Appareil électrique d'application transdermique de médicaments avec un neutralisateur

(43) Date of publication of application: 18.08.1993
(73) Proprietor: Drug Delivery Systems Inc., New York New York 10017 (US)
(72) Inventor: Sibalis, Dan, Stony Brook, N.Y. 11790 (US)
(74) Representative: Ryan, Anne Mary

(56) References cited:
- EP-A- 0 060 452
- EP-A- 0 138 347
- WO-A-85/02124

## Description

This invention relates generally to an electrical transdermal drug device delivering a drug to the patient for systemic distribution by blood flow using principles of electrokinetic phenomena, such as electrophoresis and electroosmosis, and more particularly to an electrical transdermal drug applicator delivering couteracting substances locally to the patient's skin and/or electrically inducing the skin to produce endogenous compounds which extend the period of therapeutic drug delivery and thereby increase usefulness of the drug applicator. Reference to or disclosure of devices for transdermal delivery of drugs by application of electrical current through the skin of a person or animal are shown in the following United States patents:
385 556 3 289 671 4 325 367 4 239 052
486 902 3 547 107 4 367 745 4 290 878
588 479 3 677 268 4 419 019 4 164 226
2 493 155 4 008 721 4 474 570 4 362 645
2 267 162 4 141 359 4 406 658 4 273 135
2 784 715 4 239 046 4 314 554
3 163 166 4 243 052 4 166 457

The following patents refer to or disclose transdermal drug delivery devices:
EPA No. 0060452
DE No. 2902183
DE No. 3225748
EPA No. 0058920
UK No. 2104388

EP-A-0 138 347 discloses a further iontophoretic device in which a reservoir is connected to a source of DC potential and together with the skin of a subject forms part of an electrical circuit. Means are provided for operating the electrical circuit in different operational cycles.

Thus, it is evident, that transdermal delivery of drugs by application of an electrical current is not unknown. Yet, except for experimental and developmental purposes, such electrical transdermal drug applicators are not presently commercially available for use by medical professionals or by individuals. A problem with transdermal patches, especially electrically powered patches, is that such devices exhibit a rate of drug delivery which decays with passage of time despite a steady state condition for the applied electrical current and steady state drug concentrations within the drug reservoir of the device. This phenomenon has been reported in scientific journals, for example, an article, IN VIVO TRANSDERMAL DELIVERY OF INSULIN, Chien et al, Annals of New York Academy of Sciences, pages 38-47 (1987).

Therein, changes in blood glucose level are recorded versus time after insulin is delivered transdermally to laboratory animals, using an electrical current. Several parameters are varied. For example, it is reported that a pulsed DC current has a greater and more enduring effect in reducing blood glucose levels in laboratory animals, than does a pure continuous DC current. The actual quantity of insulin, which is delivered, is not measured. Rather, the effect of the drug in reducing blood glucose levels is measured. It is found that one repetition rate of DC pulses is more effective than another pulse repetition rate in reducing blood glucose levels measured both in magnitude and time duration. A square waveform provided better results than did a sinusoidal waveform or a trapezoidal waveform.

The authors of the paper analogize the skin electrically with resistances and capacitance in parallel as an equivalent circuit. They theorize that the DC current charges the capacitance of the skin which, once charged, can accept no more current and accordingly limits drug delivery. Using DC pulses rather than steady state current allows time for the skin capacitance to discharge, such that on the next pulse, additional current, capacitor charging, and drug delivery can occur.

However, an anomalous situation arises when at a favorable pulse repetition rate, and with the same current delivery level as in prior tests, the duty cycle is varied. It would be expected that the greater the duty cycle, that is, the greater the current ON time versus the current OFF time ratio, the greater amount of insulin would be delivered transdermally and the measured effects on blood glucose level would be correspondingly more favorable and more enduring. Contrary to expectations, as the duty cycle increases from a one-to-one ratio toward an eight-to-one ratio, the reduction in blood glucose level becomes less, rather than more, although duration of this reduction is somewhat extended.

In summary, application of current over a longer period of time, that is, consumption of more energy for delivering drugs transdermally, results in what appears to be less delivery of drug as measured by the effect on blood glucose level.

That publication graphically illustrates the problem with prior art transdermal drug applicators and delivery methods using electrical current to carry drugs through the skin, that is, the effectiveness of the delivered drug is insufficient in duration of effect and the rate of drug delivery falls off as the delivering current is continuously applied over extended periods of time.

What is needed is an electrical transdermal drug applicator which provides enhanced drug delivery to the patient with regard to quantity of systemically delivered drug and duration of drug effectiveness.

### SUMMARY OF THE INVENTION

Generally speaking, in accordance with the invention as claimed, a transdermal drug patch having enhanced drug flow to the bloodstream of the subject is provided. The patch, in addition to delivering a primary drug into a subject's circulatory system for therapeutic purposes, delivers from a reservoir a non-therapeutic counteracting agent including a substance for effecting vasodilation (vasodilator) to the skin of the patient which induces flow enhancement and allows delivery of the primary drug systemically over a longer period of time and in greater quantity than heretofore appeared possible using electric current. Construction of the electrical transdermal drug patch with electrochemical flow enhancement by introduction of a counteracting agent including a substance for effecting vasodilation (vasodilator) to the skin and specific electrical wave shapes is based on applicant's appraisal of known phenomena as described above.

However, charging of skin capacitance is not considered to be the primary factor in reducing drug delivery capability as current application time and magnitude of current are increased. The efficiency of administration of insulin may be partially vitiated by adsorption and degradation within the skin tissues or by restricted blood circulation in the skin. The passage of current and/or dissociated water ions and/or certain drugs through the human skin causes a series of events related to reduction in magnitude of negative net surface charge exhibited by living mammalian cells. A most important consequence of reduction in magnitude of the negative charge on the cells is triggering of an avalanche-like coagulation process which forms thrombi, that is, blood clotting, which in turn stops blood flow through capillaries. Passage of current from a transdermal applicator tends to reduce the negative charge on cells of the skin proximate the applicator reservoir, where drugs are delivered, causing blood clotting in the capillaries which not only stops local blood flow, but also stops drug flow into the circulatory system of the subject. A drug transdermally delivered locally is not effective systemically when the capillaries of the skin contain coagulated blood.

In addition, especially in the anodic (+) region of current delivery through the skin, an immediate contraction of small blood vessels, especially arteries, takes place causing a complete interruption of blood flow to said vessels. Thus, as with blood clotting, contraction of the small blood vessels prevents drugs delivered through the skin by the transdermal applicator from being delivered into the circulatory system.

Electroosmosis_{,} which is an important factor in delivery of drugs from the applicator reservoir through the skin and into the blood circulation system, is affected by the existence of fixed negative charges on the cellular walls within the skin. A reduction of such net negative charge, as caused by passage of even small electric currents or of the water ions through the skin, inhibits electroosmosis.

Blood clotting, blood vessel contraction, and reduced electroosmotic effects, as described above, can combine synergistically to slow down or completely stop system transdermal delivery of primary drugs, especially from electrically powered transdermal applicators. This occurs even when the drug is successfully transferred from the applicator through the skin into the local skin tissue.

To counteract the current or drug induced loss of negative charge on the cellular walls within the skin, the electrical transdermal drug patch with electrochemical flow enhancement, in accordance with the invention, delivers into the skin, in addition to the primary drug having therapeutic purpose, a vasodilator and comprises electrical and/or electrochemical means for maintaining the negative surface charge density of the blood vessel walls and blood cells of the patient.

A negative charge on cell surfaces is generally accepted as a fundamental factor in preventing the clotting of blood on that surface. Negative charge (Coulombic repulsion) is also considered to be part of the mechanism for the coagulation of platelets. Additionally, overcoming negative charge is also believed to be a crucial aspect of fibrin formation, part of the avalanche of reactions in the clotting of blood (thrombosis). Without being bound by theory, for the reasons given above, it is known at a minimum, that providing a negative charge on natural or artificial surfaces in contact with animal blood helps prevent clotting or thrombosis.

One may add to the negative charge on a cell surface by reaction with or adsorption of anionic moieties compatible with animal cells such as salicylates, nitrates, methylcarboxylates_{,} sulfonates, chlorosulfonates, phosphonates, gluconates, maleates, citrates, phthalates, or sulfates. These moieties bonded to or adsorbed on a cell surface inhibit adherence of animal blood, maintain the fluidity of animal blood, and help prevent clotting of blood in motion.

Specific drugs known as anticoagulants, antiplatelets, or antifibrotics also are negatively charged and are illustrated in the Table. Among these are heparin (a mixture containing mucosaccharide sulfonates), salicylates, protamine sulfate, potassium aminobenzoate, and nitroprusside - a source of sodium nitrate. Not only are these substances direct action drugs, they are also agents for increasing the negative charge on cell surfaces and artificial surfaces in contact with animal blood. The chemicals may be chemically bonded to, adsorbed to, or absorbed in the surface.

Another class of entities for increasing the negative charge on cell surfaces or acting to inhibit either platelet formation or coagulation of animal blood are natural substances produced by the metabolism of the animal or man. Among these natural biochemical factors are: prostacyclin, thrombomodulin, Ecto-ADPase, urokinase, tissue plasminogin activators (TPA), streptokinase, antithrombin III, protein C, protein S, prostaglandins I₂ and E₁, sulfated glycosaminoglycans, N-acetylcysteine with nitroglycerin, nicoumalone, phosphatidyl inositol, hydrophilic ganglioside GM-1, cyclic GMP, S-nitrothiols, dodecapeptide gamma FlB, 400-411, and guanosine 3¹,5¹-monophosphate, their metabolic precursors and reaction products.

Additional natural vasodilators are kinins and histamines.

Such substances, if included in the reservoir of the transdermal drug delivery applicator, move through the skin and react with the cells at the same time that the primary therapeutic drug is delivered through the skin. By maintaining a more negative condition of charge on cell surfaces, blockage of flow through local blood vessels is reduced or prevented, allowing drugs delivered transdermally to be further delivered into the systemic flow. Generally, the counteractive substance including the vasodilator has no therapeutic value, although in special instances a substance may serve a dual purpose.

Maintaining a more negative condition of charge on cell surfaces could be achieved simultaneously and/or alternatively by precharging the cell surface with a negative charge prior to electroosmotic drug delivery in cases where such delivery takes place from the positive drug reservoir. The precharging and the discharging voltage levels are monitored and maintained within preset limits by electronic means.

In situations where one polarity of voltage delivers the primary drug and the opposite polarity delivers the counteractive substance including the vasodilator, arrangements can be made for simultaneous delivery of both substances, or alternatively, alternating delivery can be provided.

To prevent formation of thrombi of platelets, that is, blood coagulation, adjacent the applicator/skin interface, antithrombotic agents may be delivered from the applicator reservoir, either as a preconditioner or during drug delivery or alternately. Such additional counteractive substances would include, for example, heparin or aspirin. To counteract contraction or constriction of blood vessels adjacent the applicator interface, vasodilators are used in the reservoir. Nitroglycerin is one such dilator. Concomitantly, specific electrical pulses, such as square wave pulses of 0.4 ms and a frequency of 80 Hz at an intensity which could produce a tingling sensation may be used for repetitive periods of up to two hours a day to maintain vasodilation.

Accordingly, it is an object of the invention to provide an improved transdermal drug applicator which provides enhancement of drug flow into the system of the subject according to claim 1.

Still other objects and advantages of the invention will in part be obvious and will in part be apparent from the specification.

### BRIEF DESCRIPTION OF THE DRAWINGS

For a fuller understanding of the invention, reference is had to the following description taken in connection with the accompanying drawings, in which:
Figure 1 is a cross-section of human skin showing pathways for transdermal drug delivery.
Figure 2 is an electrical transdermal drug patch in accordance with the invention including a single reservoir holding both a primary drug and a counteractor including a vasodilator.
Figure 3 is an electrical transdermal drug patch in accordance with the invention including a parallel arrangement of reservoirs; one holding a primary drug; one a vasodilator; the other holding an additional counteractor.
Figure 4 is an electrical transdermal drug patch in accordance with the invention including two reservoirs electrically in series.
Figures 5-8 illustrate alternative arrangements of reservoirs and circuitry in accordance with the invention.

### DESCRIPTION OF THE PREFERRED EMBODIMENTS

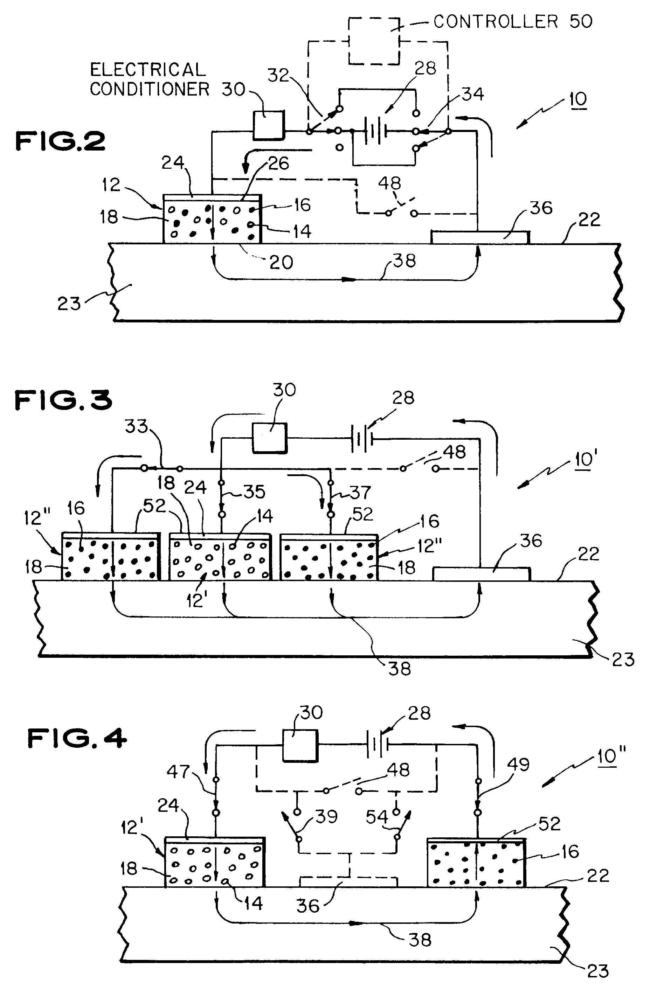

With reference to the Figure 2, an electrical transdermal drug patch 10 in accordance with the invention includes a reservoir 12 containing a primary drug 14 and a counteractor 16 including a vasodilator, both being dispersed in a suspension, for example, a gel 18 as disclosed in any of the above-referenced patents by the inventor here (as examples). A surface 20 of the reservoir 12 rests against the surface 22 of the user's skin 23 and is maintained in position, for example, by an adhesive (not shown). An electrode 24 connects to another surface 26 of the reservoir 12 and this in turn is connected to a DC source 28 by way of an electrical current conditioner 30 and a single pole switch 32. The other terminal of the DC source 28 connects to the skin surface 22 by way of a return electrode 36 which directly contacts the skin and is maintained in position, for example, by an adhesive (not shown). A single pole switch 34 is intermediate the electrode 36 and the DC source 28.

As discussed more fully hereinafter, the counteractor 16 including the vasodilator acts locally on the blood vessels, for example, blood capillaries, whereas, as described in the Sibalis patents cited above, the primary drug is delivered systemically into the body's circulatory system.

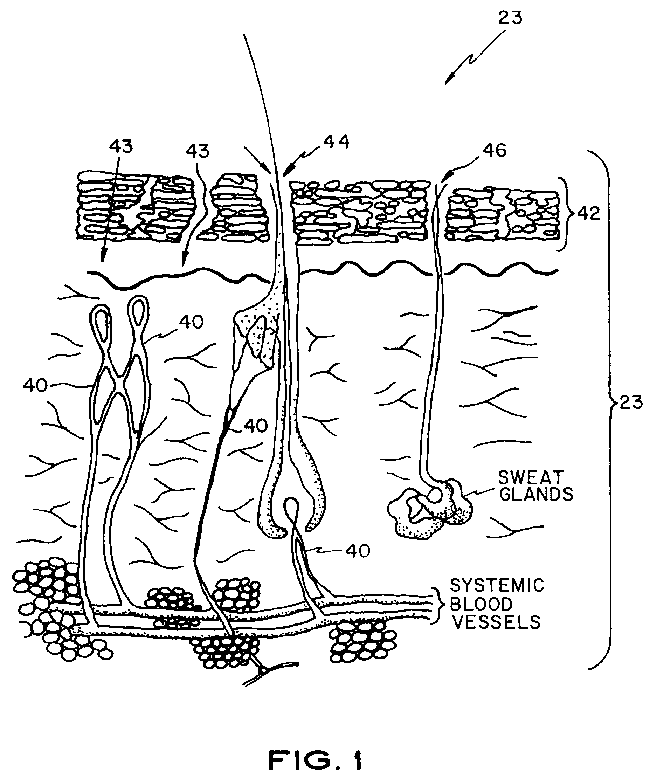

It should be understood that the skin 23 is illustrated in Figures 1, 2 with simplified representations and the electrical transdermal drug applicator is also shown schematically in Figure 2 as a generic representation of such a device. More detailed descriptions may be found in the above-cited references by the inventor in this application. It suffices here to state that the gel 18 and the primary drug 14 and counteractor 16 including the vasodilator are contained in the reservoir 12 in a manner to prevent leakage of the substances. Also, there is no short-circuit of electrical current across the skin surface 22 directly to the electrode 36.

When the switches 32, 34 are closed as illustrated, a positive potential appears on the electrode 24 and a negative potential on the electrode 36 causing a current to flow from the source 28 through the current conditioner 30, the electrode 24, reservoir 12 and skin surface 22 in series. A DC current flows within the skin 23 as indicated by the arrow 38, then back through the skin surface 22 to the return electrode 36, and then back through switch 34 to the negative terminal of the DC source 28. The positive potential of the source 28 applied to the electrode 24 and the electrical current drive the primary drug 14 and the counteractor 16 through the interface between the reservoir 12 and the skin surface 22.

In Figure 1, the human skin 23 is represented in simplified construction as including an outer layer, the epidermis 42, which is broken by hair follicles 44 and sweat ducts 46, and at greater depth blood capillaries 40, glands, etc. In electrical transdermal drug applicators, it is known that small quantities of the drug pass directly through the epidermis 42 as indicated by the arrows 43 but also the drug 14 enters the skin with relative ease through the hair follicles 44 and sweat ducts 46 which act as shunts. Having entered into the skin, the drug 14 is disseminated to the systemic circulatory system by electrokinetic processes, for example, electrophoresis, electroosmosis, iontophoresis, etc. With certain drugs and counteractors including the vasodilator it may be desirable to pick skin areas with greater or lesser densities of hair follicles and sweat ducts for application of the transdermal patch 10 thereto.

Where a reversed polarity from that described in Figure 2 is required to drive the primary drug 14 and counteractor 16 including the vasodilator into the skin tissues, the switches 32, 34 in Figure 2 are moved to the positions indicated with broken lines, whereby a negative potential is applied to the electrode 24 and a positive potential is applied to the return electrode 36.

Where the primary drug 14 and the counteractor 16 including the vasodilator require opposite polarities of voltage to cause the substances to enter into the skin, an alternating DC potential is applied by periodically changing the positions of the switches 32, 34 such that the potentials on the electrodes 24, 36 are periodically reversed. The timing of the switches 32, 34 in each alternating position is based upon the drug 14 and counteractor 16 which are being used. Equal driving times or unequal driving times can be provided as best suited for the substances 14, 16.

Further, in recognition of the work reported by Chien et al as discussed above, it may be desirable, in the process of drug delivery, to incorporate time periods wherein no potential is applied to the electrodes 24, 36 and, it may be desirable during those periods of no driving potential, to apply a short circuit between the electrodes 24, 36 such that charges, if any, built up within the skin during the driving periods may be readily discharged. The switch 48, shown with broken lines in Figure 2, is connected between the electrodes 24, 36 and when closed provides the desired short circuit.

In electrical transdermal drug patches in accordance with the invention, wherein a complex operational cycle is desirable, including (for examples) polarity reversals, periods without driving potential, periods of electrode short-circuiting, etcetera, a controller 50, also shown in broken lines in Figure 2, is used to automatically regulate opening and closing of the switches 32-35, 37, 48 in desired programs.

It should be understood that whereas the power source 28 is indicated in Figure 2, and in the other Figures for the sake of illustration, as a DC battery, the power source may include circuitry for converting potential from a DC battery to voltages of controlled magnitude with regulated current delivery; the electrodes not being connected directly to the DC battery but to the output of the voltage generating circuitry. Additionally, the switches which are schematically represented in Figure 2 as electro-mechanical switches can be solid state switches, especially when considering the very low current flows which are frequently involved in electrical transdermal drug applicators as indicated in the patents of the present inventor cited above. Thus complex operational cycles of an applicator in accordance with the invention may be automatically controlled by a microchip.

The counteractors 16 including a vasodilator, which are added to the reservoir 12, can operate within the skin to accomplish one or more effects which tend to maintain blood circulation in the skin area adjacent the transdermal drug patch 10, such that the primary drug 14, which enters the skin, is carried away by the bloodstream into systemic circulation for therapeutic purposes. The counteractors 16 include vasodilators which operate by relaxing the muscles surrounding the blood vessel walls, including capillary walls, such that a greater flow area and easier blood flow is possible. An additional counteractor 16 may fall in the category of antithrombin agents in that they work to reduce platelet accumulation and blood clotting in the blood vessels, in particular the capillaries, in the area where the drug patch 10 is applied. A single additional counteractor which performs both functions may be used in the reservoir 12 or a plurality of counteractors which in combination perform both functions, or a counteractor which performs only one such function may be used in the reservoir 12. Also, the additional counteractor 16 may be a substance which when introduced into the skin induces the body to produce substances which delay, inhibit, or eliminate blood coagulation or aid in dilating the blood vessels to improve blood circulation. The primary drug and counteractor including the vasodilator may be variants of the same substance which do not interact pharmacologically, e.g. nitroglycerine and isosorbide dinitrate. The counteractive substance including the vasodilator can be part of the primary drug molecule. Especially sulfonated, phosphorylated and carboxylated groups attached to the primary drug molecules may be effective in providing a desired increase of the negative charge characteristics of the cellular walls where the patch is attached.

Substances known to be unsuitable for systemic transdermal delivery as a primary drug with intended therapeutic benefit may be the preferred counteractor including the vasodilator as the drug's action will be limited to the target area of patch attachment and the counteractor including the vasodilator will not be available to produce any systemic effects. Thus, such application of substances as counteractors including the vasodilator is entirely opposed to prior teachings where it may be indicated that no therapeutic utility for these materials is present in transdermal applicators The counteractors including the vasodilator are formulated to function only as topical agents. For example, if nitrates are used, e.g. nitroglycerin, the flux rate of the counteractor including the vasodilator may be adjusted so as not to produce any detectable blood serum level of the counteractor substance including the vasodilator, while at the applicator site blood circulation is improved. There is no or negligible systemic effect or pharmacological effect. More specifically, the counteractive substance including the vasodilator will be formulated for negligible transdermal delivery when its use is limited only to the counteractive function. The counteractive substance including the vasodilator could be of a nature which selectively allows its delivery through the stratus corneum, such as nitroglycerin, whereas the electrokinetic main drug delivery takes place via the skin shunts, perspiration and sebaceous ducts. In such a case the stratum corneum would function as a depot for the counteractive substance including the vasodilator even though the counteractive substance including the vasodilator previously contained in said applicator reservoir is exhausted from the applicator.

Known vasodilators which may be used as vasodilator, and known antithrombin substances which may be used as additional counteractors and substances which may serve as both blood vessel dilators and also act to reduce or eliminate blood coagulation, are set forth herein below.

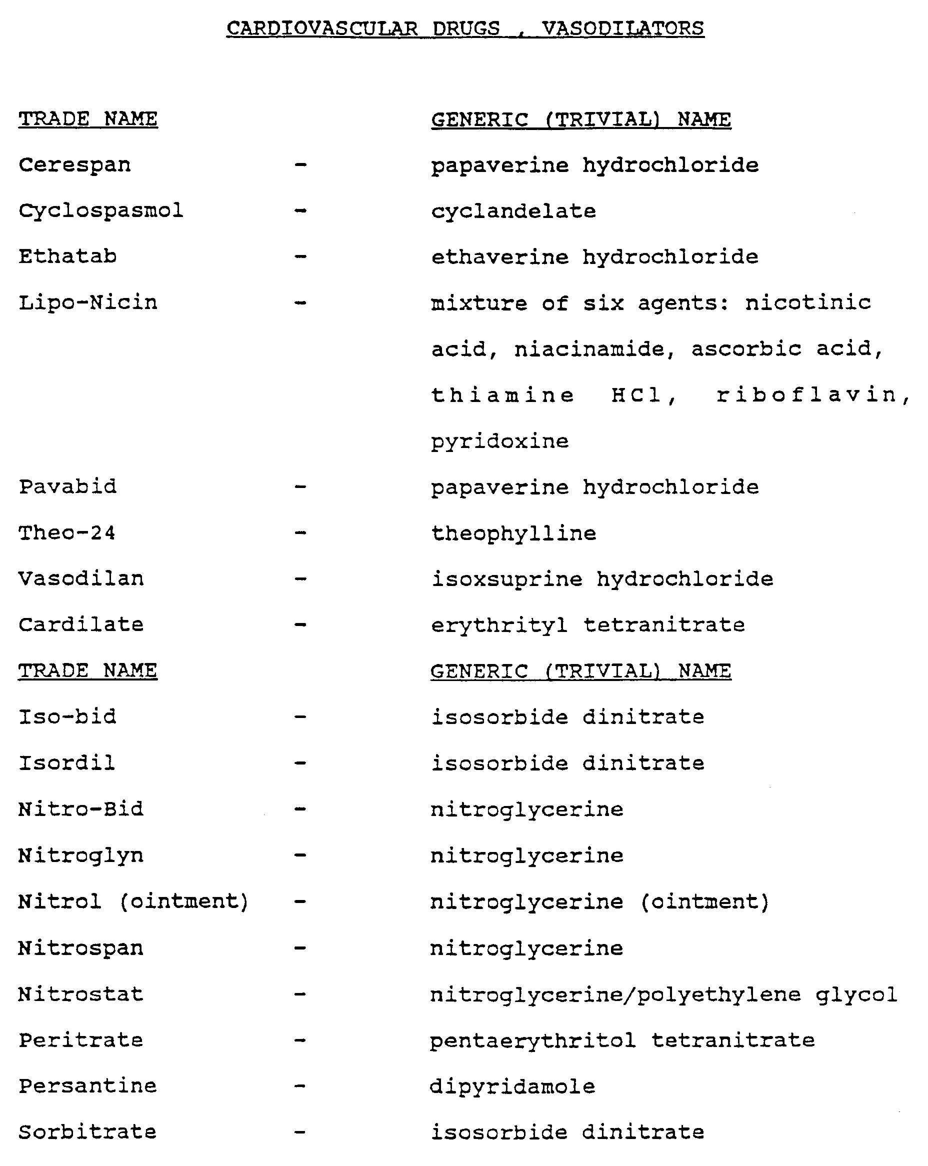

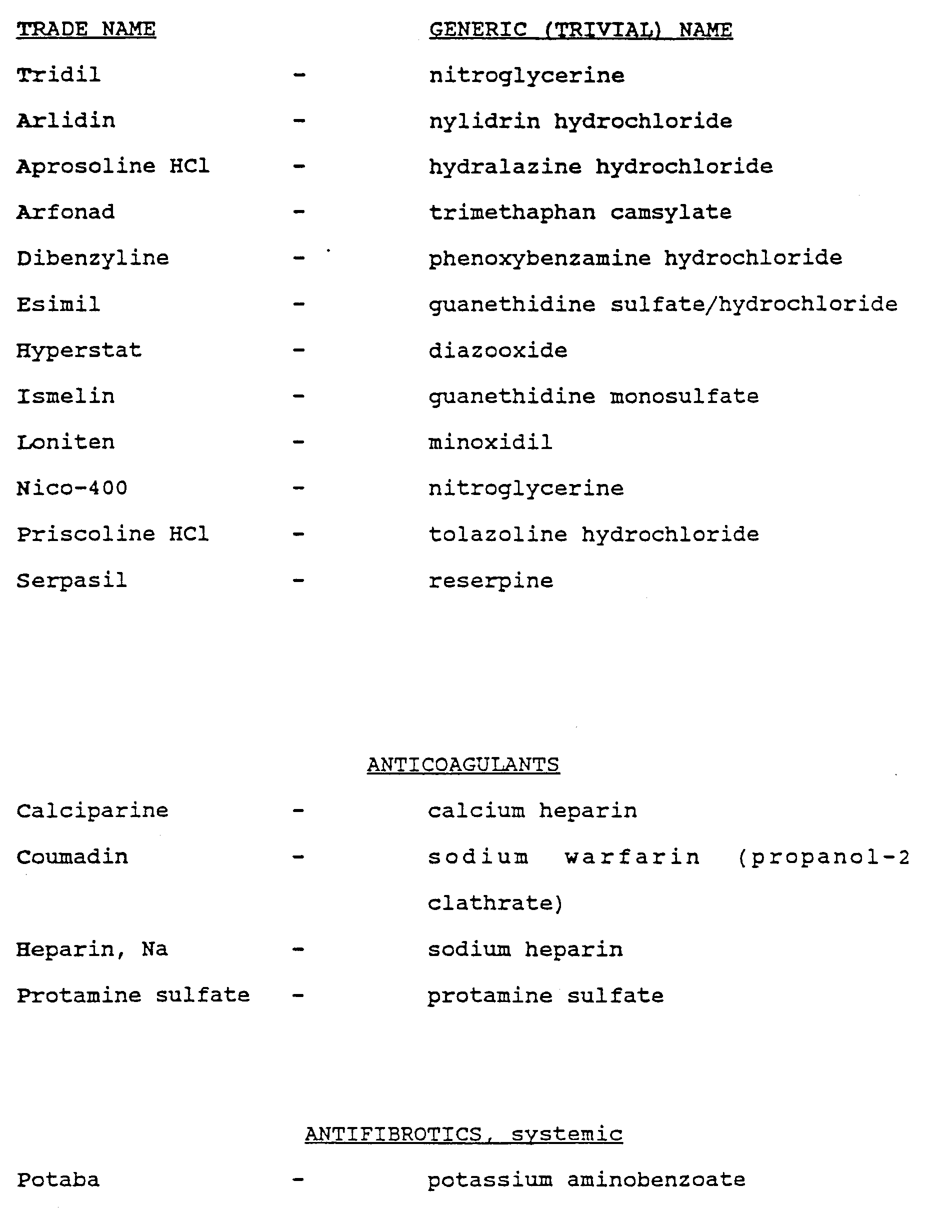

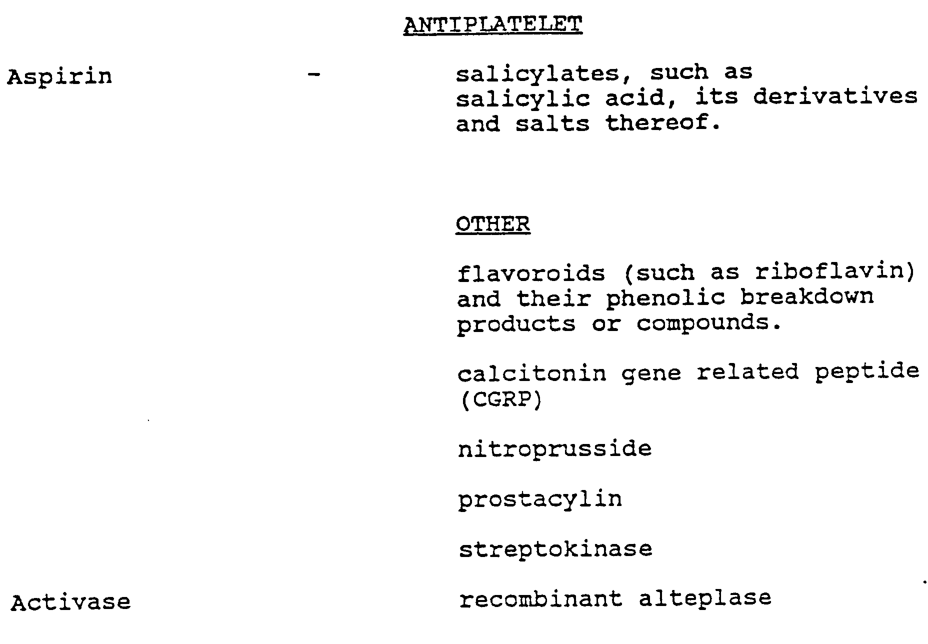

While the above listings are by no means complete, they are nevertheless representative of various categories of drugs or agents which may be suitable in the practice of the invention. Moreover, the present invention contemplates the use of any counteractors including a vasodilator which have the specific characteristics and produce the effects desired as have been described in the present discription.

Figure 3 illustrates an alternative embodiment of an electrical transdermal drug applicator 10' in accordance with the invention, wherein the primary drug 14 is contained in a first reservoir 12' and the counteractor 16 including a vasodilator is contained in the reservoirs 12''. In each reservoir, the substances are suspended in a gel 18. Electrodes 24, 52 are connected in parallel to receive current from the DC power source 28 by way of the electrical current conditioner 30. As illustrated, current flows from the battery 28 through the current conditioner 30 to the electrodes 24, 52, through the associated reservoirs 12', 12'' and through the surface 22 of the skin 23. The current flows (arrows 38) within the skin to the return electrode 36 and then back to the DC power source 28.

To suit a particular primary drug 14 and counteractor 16 including the vasodilator, provision for switching the polarity of the DC source 28 may be provided as indicated in Figure 2, and a shorting circuit connecting all electrodes 24, 52 directly to the return electrode 36 by way of a switch 48 may also be included. By operation of switches 33, 35, 37, reservoirs may be selectively inactivated while the other reservoirs continue to function. A controller 50 may be used to control the switches 32, 34, 48 (see Figure 2) when periodic cycling is involved in operation of the transdermal drug applicator 10'.

Figure 4 illustrates another alternative embodiment of an electrical transdermal drug applicator 10''. In this configuration, the reservoir 12' is connected to one terminal of the DC source 28, whereas the reservoir 12'' is connected to the other terminal of the DC power source 28. Thereby, opposite polarities are always present on the two reservoirs 12', 12''. This is advantageous when the primary drug 14 is delivered through the skin's surface 22 by one electrical potential and the counteractor 16 including the vasodilator is delivered through the skin surface 22 by the opposite potential. In this way, both the drug 14 and counteractor 16 including the vasodilator can be continuously and concurrently delivered if desired.

A shorting circuit between the electrodes 24, 52 including the switch 48 may be used to remove charge, if any, from the skin during periods when the voltage is not applied. A controller 50 may be used with the configuration of Figure 4 as described above to control ON/OFF periods, periods when the short circuit through the switch 48 is desired, etc. A return electrode 36 (broken lines) may be used to eliminate the reservoir 12' from the circuit when a switch 39 is closed while switches 47, 54 are open while switch 48 is also open. In this way, delivery of the counteractor substance 16 including the vasodilator to the skin may continue while delivery from the reservoir 12' of the primary drug 14 is discontinued. Similarly, the return electrode 36 can be used to eliminate counteractor reservoir 12'' when it is desired to deliver the drug 14 while interrupting delivery of the counteractor 16 including the vasodilator. In this case the switch 47 is closed, switches 38, 48, 49 are open and switch 54 is closed.

The electrical transdermal drug patch 10 of Figure 2 was described as containing a drug and a counteractor including the vasodilator in suspension, for example, a gel. However, it should be understood that in alternative embodiments of an electrical transdermal drug applicator in accordance with the invention, the reservoir may be in the form of a matrix, liquid, paste, etc. as suits the particular substances in use. Also, Figure 2 illustrates a generally random and equal distribution of drug 14 and counteractor 16 including the vasodilator within the reservoir 12. It should be understood that the reservoir may contain a predominance of one substance over the other. The distribution of materials may not be uniform or randomized. The drug 14 may be in one layer, whereas a counteractor 16 including the vasodilator may be in another layer, the layers being at different distances from the skin surface 22. As dictated by the particular application, either the drug 14 or counteractor 16 layer including the vasodilator may be closer to the skin surface. Such layers may themselves combine several substances which can be in varying proportions as suits the particular construction with drug 14 and counteractor 16 in each layer. The layers may be of different thicknesses such that one layer may act as a flow inhibitor of materials from the other layer. There may be several layers each of counteractors and drugs and these layers may be alternated in their stacking within a reservoir.

In an exemplary embodiment of the invention, blockage of the capillaries and stratum corneum of the skin is avoided or inhibited by sub-therapeutic dosages of an active vasodilator such as nitroglycerin. For example, a therapeutic ointment at 2% concentration is available from the W.H. Rorer Co. (Fort Washington, Penna 19034) under the tradename NITROL ointment. Since it is known that nitroglycerine relaxes smooth muscles, principally in the smaller blood vessels thus dilating arterioles and capillaries, it may be advantageous to topically apply sub-therapeutic doses of about 0.001 to about 0.2% nitroglycerine ointments to the skin, when employing an otherwise conventional transdermal applicator, such as described in the applicant's own earlier issued U.S. Patents. Thus a counteractor layer including the vasodilator is at the skin surface; the primary drug passes through this layer before entering the skin. If desired, the body of the ointment may preferably be a hydrophilic polymer, such as polyvinyl pyrrolidone or neutralized polyacrylic acid and the like in order not to interfere with the hydrophilic adhesive which may be employed in the transdermal patch.

In another exemplary embodiment, one utilizes a stabilized vasodilator in order to restrict blockage counteraction to the region of the patient's body where the transdermal patch of the invention is located. This may be achieved by the use of a polymer stabilizer for a vasodilator, such as nitroglycerine. One such polymer stabilizer is polyethylene glycol, but other stabilizers having like properties may also be suitable in the practice of the invention. As is known, a polyethylene glycol of molecular weight 3350 operates to lower the migration of nitroglycerine, (see U.S. Patent 3,789,119). With the present invention, a higher molecular weight would be preferred, for example of from about 5000 to about 20,000 so as to localize the vasodilation of the very region where the electrolytic patch of the invention is applied. Of course, other suitable benign polymers with a molecular weight of from abut 3,000 to about 30,000 may be employed, depending on their diffusion constant.

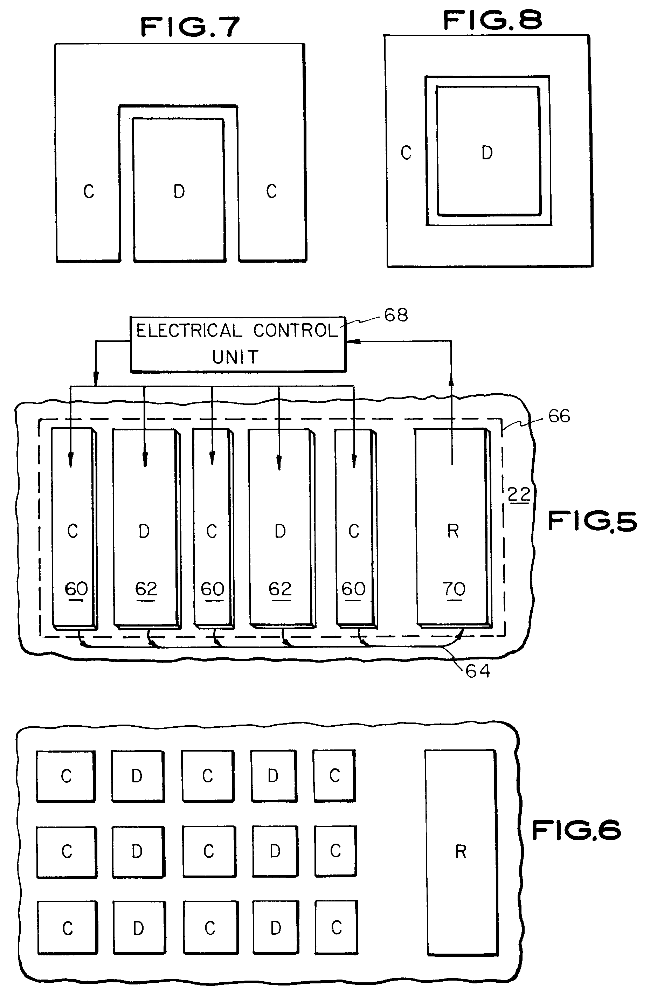

Figures 5-8 illustrate alternative embodiments in accordance with the invention wherein the primary drug indicated in those Figures with a D and the counteractive agent including the vasodilator, indicated in those Figures with a C, are located in individual reservoirs. In Figure 5, counteractor reservoirs 60 alternate with drug reservoirs 62 in the direction of current flow indicated by the arrows 64 when a patch 66 is attached to the skin surface 22. For the sake of example, the reservoirs are connected in parallel schematically to one terminal of an electrical control unit 68 and current flows from the reservoirs 60, 62 through the skin surface 22 and within the skin to the return electrode 70 indicated in Figure 5 by the letter R. The skin to which the patch 66 is attached receives the primary drug from the reservoirs 62, while at the same time a current passing through a counteractor reservoir 60 from the upstream direction (left to right in Figure 5) delivers the counteractive substance including the vasodilator and acts as a preconditioner to the blood vessels in the area of the drug reservoirs 62. The counteractive substance including the vasodilator and the primary drug are thereby simultaneously active in the same region of skin.

The Figures 5-8 are schematic. Any electrical control, such as polarity reversal, ON/OFF voltage application, electrode short-circuiting, series arrangement of reservoirs, etc., as described above in relation to Figures 2-4, can be applied equally to the arrangements of Figures 5-8. Figures 6, 7 and 8 show applicators with separate drug D reservoirs and counteractor C reservoirs. In each instance, the reservoirs may be electrically connected such that the counteractor substance including the vasodilator acts as a preconditioner for the blood vessels in the area where the drug reservoir is applied. With regard to Figures 5-8, it should be understood that the relative positions of the primary drug reservoirs D may be interchanged with the counteractive substance reservoirs C as suits the particular substances which are in use.

It will thus be seen that the object set forth above, among those made apparent from the preceding description, are efficiently attained and, since certain changes may be made in the construction set forth without departing from the invention as claimed, it is intended that all matter contained in the above description and shown in the accompanying drawings, shall be interpreted as illustrative and not in a limiting sense.

## Claims

1. A transdermal drug patch (10) for delivering at least one drug to the bloodstream of a patient over an extended period of time comprising:
(a) reservoir means (12) for containing one or more therapeutic drugs (14) to be delivered to the bloodstream of a patient through the skin (22, 23) thereof;
(b) a source (28) of DC potential having a return electrode (36) connected to one polarity terminal thereof and said reservoir means (12) connected to an opposite polarity terminal thereof forming an electrical series circuit when the reservoir means (12) and return electrode (36) are applied to the skin (22, 23) of the patient and interface therewith:
(c) means (30, 32, 34, 48, 50) for operating the electrical circuit in different operational cycles to effect application of the therapeutic drug or drugs (14) into the bloodstream of the patient through the skin (22, 23) by electrokinetic processes including electrophoresis and electroosmosis depending on the drug (14) to be administered to the patient:
(d) characterised in that the patch (10) further comprises electrical and/or electrochemical means for maintaining the negative surface charge density of the blood vessel walls and blood cells of the patient at least in a region where the transdermal patch is attached to the skin (22, 23) of the patient; and
(e) in that said reservoir means (12) contains a substance (16) for delivery to the bloodstream of the patient for effecting vasodilation .

2. A transdermal drug patch (10) according to claim 1, characterized in that said means (30, 32, 34, 48, 50) for operating the electrical circuit in different operational cycles comprises an electrical current conditioner (30) connected between said source (28) and said reservoir means (12) in series therewith.

3. A transdermal drug patch (10) according to any proceding claim, characterized in that said means (30, 32, 34, 48, 50) for operating the electrical circuit in different operational cycles comprises switching means (32, 34, 50) for effecting polarity reversals.

4. A transdermal drug patch (10) according to any proceeding claim, characterized in that said means (30, 32, 34, 48, 50) for operating the electrical circuit in different operational cycles comprises means (48, 50) for varying periods in which the circuit is free of electrical potential during an operational cycle.

5. A transdermal drug patch (10) according to claim 1, characterized in that said means (30, 32, 34, 48, 50) for operating the electrical circuit in different operational cycles comprises means (48, 50) for selectively effecting a shortcircuit between said reservoir means (12) and said return electrode (36).

6. A transdermal drug patch (10) according to any preceding claim, characterized in that said electrical and/or electrochemical means comprises electrical means, forming part of said electrical circuit, for precharging the cell surface with a negative charge prior to electrophoretic or electroosmotic delivery, and wherein said reservoir means (12) is connected to a positive terminal of said DC source (28).

7. A transdermal drug patch (10) according to any preceding claim, characterized in that said electrical and/or electrochemical means comprises electrical means, forming part of said electrical circuit, for generating electrical pulses effective to maintain vasodilation.

8. A transdermal drug patch (10) according to claims 7, characterized in that said pulses are square wave pulses of 0.4 ms and a frequency of 80 Hz.

9. A transdermal drug patch (10) according to claim 1, wherein the reservoir means (12', 12'') comprises a first reservoir (12') and a second reservoir (12''), the first reservoir (12') containing a primary drug intended for therapeutic purposes and the second reservoir (12'') containing said substance (16) for effecting vasodilation.

## Patentansprüche

1. Transdermaler Medikamentenapplikator (10) zum Zuführen mindestens eines einzelnen Medikaments in den Blutstrom eines Patienten während einer ausgedehnten Zeitdauer, aufweisend:
(a) eine Reservoireinrichtung (12) zum Enthalten eines oder mehrerer therapeutsicher Medikamente (14), die dem Blutstrom eines Patienten durch die Haut (2, 23) desselben zugeführt werden sollen;
(b) eine Quelle (28) mit Gleichstrompotential, die eine Rückleitungselektrode (36)aufweist` welche mit einer Polaritätsanschlußklemme verbunden ist, während die Reservorieinrichtung mit der entgegengesetzten Polantätsanschlußklemme verbunden ist, und die eine elektrische Reihenschaltung bilden, wenn die Reservoireinrichtung (12) und die Rückleitungselektrode (36) an die Haut (22, 23) des Patienten angelegt werden und Übergangsstellen mit ihr bilden;
(c) Einrichtungen (30, 32, 34, 48, 50) zum Betreiben der elektrischen Schaltung in unterschiedlichen Betriebzyklen, um das Zuführen des therapeutischen Medikaments bzw. der Medikamente (14) in den Blutstrom des Patienten durch die Haut (22, 23) mittels elektrokinetischer Prozesse unter Einschluß der Elektrophorese und der Elektroosmose zu bewirken, je nach dem Medikament (14), das dem Patienten verabreicht werden soll;
(d) dadurch gekennzeichnet, daß der Applikator (10) weiter elektrische und/oder elektrochemische Mittel zum Aufrechterhalten der negativen Oberflächenladungsdichte der Blutgefäßwände und Blutzellen des Patienten mindestens in einem Bereich aufweist, in welchem der transdermale Applikator auf der Haut (22, 23) des Patienten befestigt wird; und
(e) daß die Reservoireinrichtung (12) eine, dem Blustrom des Patienten zuzuführende Substanz (16) zur Durchführung einer Gefäßerweiterung enthält.

2. Transdermalar Medikamentenapplikator (10) nach Anspruch 1, dadurch gekennzeichnet, daß Einrichtungen (30, 32, 34, 48, 50) zum Betreiben der elektrischen Schaltung im unterschiedlichen Betriebszyklen einen elektrischen Stromkonditionerer (30) umfassen, der zwischen die Quelle (28) und die Reservoireinrichtung (12) in Reihe geschaltet ist.

3. Transdermaler Medikamentenapplikator (10) nach einem der vorhergehenden Ansprüche, dadurch gekennzeichnet, daß die Einrichtungen (30, 32, 34, 48, 50) zum Betreiben der elektrischen Schaltung in unterschiedlichen Betriebszyklen Schalteinrichtungen (32, 34, 50) zum Bewirken von Polaritätsumkehrungen umfassen.

4. Transdermaler Medikamentenapplikater (10) nach einem der vorhergehenden Ansprüche, dadurch gekennzeichnet, daß die Einrichtungen (30, 32, 34, 48, 50) zum Betreiben der elektrischen Schaltung in unterschiedlichen Betriebszyklen Einrichtungen (48, 50) zum Variieren von Perioden umfassen in denen die Schaltung während eines Betriebszyklus' elektrisch potentialfrei ist.

5. Transdermaler Medikamentenapplikator (10) nach Anspruch 1, dadurch gekennzeichnet, daß die Einrichtungen (30, 32, 34, 48, 50) zum Betreiben der elektrischen Schaltungen in unterschiedlichen Betriebszyklen Einrichtungen (48, 50) zum selektiven Bewirken eines Kurzschlusses zwischen der Reservoireinrichtung (12) und der Rückleitungselektrode (36) umfassen.

6. Transdermaler Medikamentenapplikator (10) nach einem der vorhergehenden Ansprüche, dadurch gekennzeichnet, daß die elektrischen und/oder elektrochemischen Mittel elektrische Mittel umfassen, die Bestandteile der elektrischen Schaltun sind, um die Zelloberfläche vor der elektrophoretischen oder elektroosmotischen Zufuhr mit einer negativen Ladung vorzuladen, und bei dem die Reservoireinrichtung (12) an eine positive Anschlußklemme der Gleichstromquelle (28) angeschlossen ist.

7. Transdermaler Medikamentenapplikator (10) nach einem der vorhergehenden Ansprüche, dadurch gekennzeichnet, daß die elektrischen und/oder elektrochemischen Mittel elektrische Einrichtungen umfassen, die Bestandteil der elektrischen Schaltung sind, um elektrische Impulse zu erzeugen, die zum Aufrechterhalten der Gefäßerweiterung wirksam sind.

8. Transdermaler Medikamentenapplikator (10) nach Anspruch 7, dadurch gekennzeichnet, daß die Impulse Rechteckwellenimpulse von 0,4 ms und einer Frequenz von 80 Hz sind.

9. Transdermaler Medikarmentenapplikator (10) nach Anspruch 1, bei dem die Reservoireinrichtungen (12', 12'')ein erstes Reservoir (12') und ein zweites Reservoir (12'') umfassen, wobei das erste Reservoir (12') ein primäres Medikamt für therapeutische Zwecke enthält und das zweite Reservoir (12'') die Substanz (16) zum Bewirken der Gefäßerweiterung enthält.

## Revendications

1. Patch de médicament transdermique (10) destiné à l'administration d'au moins un médicament dans la circulation sanguine d'un patient pendant une durée prolongée, comprenant :
(a) un moyen de réservoir (12) destiné à contenir un ou plusieurs médicaments (14) qui doivent être administrés dans la circulation sanguine d'un patient à travers la peau (22, 23) de celui-ci;
(b) une source (28) de courant continu ayant une électrode de retour (36), reliée à une borne de la source, et ledit moyen de réservoir (12), relié à la borne de polarité opposée, formant un circuit électrique en série lorsque le moyen de réservoir (12) et l'électrode de retour (36) sont appliquées à la peau (22, 23) du patient et sont en contact interfacial avec celle-ci ;
(c) un moyen (30, 32, 34, 48, 50) destiné à commander le circuit électrique dans différents cycles de fonctionnement pour effectuer l'administration du ou des médicaments (14) dans la circulation sanguine du patient à travers la peau (22, 23) par un processus électrocinétique comprenant l'électrophorèse et l'électro-osmose, en fonction du médicament (14) qui doit être administré au patient;
(d) caractérisé par le fait que le patch (10) comprend en outre des moyens électriques et/ou électrochimiques pour maintenir la densité de charge à la surface des parois des vaisseaux sanguins et des cellules sanguines du patient à une valeur négative, au moins dans la région où le patch transdermique est fixé sur la peau (22, 23) du patient; et
(e) que ledit moyen de réservoir (12) contient une substance (16) destinée à être administrée dans la circulation sanguine du patient afin d'y produire une vasodilatation.

2. Patch de médicament transdermique (10) selon la revendication 1, caractérisé par le fait que ledit moyen (30, 32, 34, 48, 50) destiné à commander le circuit électrique dans différents cycles de fonctionnement comprend un régulateur (30) de courant électrique monté en série entre ladite source (28) et ledit moyen de réservoir (12).

3. Patch de médicament transdermique (10) selon l'une quelconque des revendications précédentes. caractérisé par le fait que ledit moyen (30, 32, 34, 48, 50) destiné à commander le circuit électrique dans différents cycles de fonctionnement comprend un commutateur (32, 34, 50) destiné à effectuer l'inversion de polarité.

4. Patch de médicament transdermique (10) selon l'une quelconque des revendications précédentes, caractérisé par le fait que ledit moyen (30, 32, 34, 48, 50) destiné à commander le circuit électrique dans différents cycles de fonctionnement comprend un moyen (48, 50) destiné à faire varier les périodes pendant lesquelles le circuit est hors tension au cours du cycle de fonctionnement.

5. Patch de médicament transdermique (10) selon l'une quelconque des revendications précédentes. caractérisé par le fait que ledit moyen (30, 32, 34, 48, 50) destiné à commander le circuit électrique dans différents cycles de fonctionnement comprend un moyen (48, 50) destiné à réaliser sélectivement un court-circuit entre le moyen de réservoir (12) et ladite électrode de retour (36).

6. Patch de médicament transdermique (10) selon l'une quelconque des revendications précédentes. caractérisé par le fait que ledit moyen électrique et/ou électrochimique comprend des moyens électriques qui font partie dudit circuit électrique, destinés à charger préalablement la surface cellulaire avec une charge négative avant l'administration électrophorétique ou électro-osmotique. et par le fait que ledit moyen de réservoir (12) est raccordé à la borne positive de ladite source de courant continu (28).

7. Patch de médicament transdermique (10) selon l'une quelconque des revendications précédentes. caractérisé par le fait que ledit moyen électrique et/ou électrochimique comprend des moyens électriques qui font partie dudit circuit électrique. destinés à générer des impulsions électriques efficaces pour maintenir une vasodilatation.

8. Patch de médicament transdermique (10) selon la revendication 7, caractérisé par le fait que lesdites impulsions sont des impulsions d'ondes carrées de 0.4 ms ayant une fréquence de 80 Hz.

9. Patch de médicament transdermique (10) selon la revendication 1, dans lequel le moyen de réservoir (12', 12'') comprend un premier réservoir (12') et un second réservoir (12''), le premier réservoir (12') contenant un premier médicament destiné à des fins thérapeutiques et un second réservoir (12'') contenant ladite substance (16) destinée à produire une vasodilatation.
